# EUROPEAN PATENT APPLICATION

(11) **EP 3 919 053 A1**
(43) Date of publication of application: **08.12.2021**
(21) Application number: 21180324.2
(22) Date of filing: 30.09.2014
(51) Int. Cl.: A61K 31/14, A61K 31/202, A61K 31/355, A61K 31/375, A61K 45/06, A61K 31/4415, A61K 31/519, A61K 31/7068, A61K 31/7072, A61K 31/714, A61K 33/04, A61P 25/02, A61P 25/14, A61P 25/16, A61P 25/28, A23L 33/10, A61P 1/10

(54) **COMPOSITION COMPRISING A URIDINE SOURCE, AN OMEGA-3 PUFA FOR IMPROVING COORDINATION, BALANCE, GRIP STRENGTH OR FINE MOTOR SKILLS**

(30) Priority: 06.12.2013 WO PCT/NL2013/050879
(62) Divisional of application: 14815077.4
(71) Applicant: N.V. Nutricia, 2712 HM Zoetermeer (NL)
(72) Inventor: ATTALI, Amos, 2712 HM Zoetermeer (NL)
(74) Representative: V.O.

(57) **Abstract**

The invention is directed to a combination of a uridine source and an omega-3-polyunsaturated fatty acid having 18-24 carbon atoms for use in
- the prevention or treatment of a disturbance in coordination of limbs in a mammal
- the prevention or treatment of a disturbance in equilibrium in a mammal
- for use in the prevention or treatment of a disturbance in limb strengthA combination of a uridine source and an omega-3-polyunsaturated fatty acid having 18-24 carbon atoms
- or use in the prevention or treatment of a disturbance in a motor skill in a mammal.

## Description

The invention relates to a composition comprising a uridine source, an omega-3 polyunsaturated fatty acid for use in the improvement of a subject's coordination, balance, grip strength or fine motor skills.

The nervous system, in particular the brain, plays an essential role in a mammal's life. For example cognitive, emotional, social, sensory, motoric and regulatory functions are mainly determined by the brain. In society more and more diseases, disorders and problems with a proper functioning of the brain are becoming recognised. Brain functioning can become impaired, e.g. due to traumata (e.g. accidents, violence), abuse of drugs (e.g. alcohol) or systematic malnourishment. Also during aging several deleterious changes occur in the nervous system and in particular in the brain. Malfunctioning of the brain is therefore especially a problem in the elderly and more in particular in the frail or malnourished elderly. In this group, several diseases and disorders which are associated with or find their cause in a badly functioning nervous system are therefore relatively frequently observed. Examples are several forms of dementia, progressive neurodegenerative disorders, mood disorders but also other diseases or disorders like abnormal behaviour.

Several nutritional approaches have been proposed in the art to support brain function. For instance, WO 2009/002165 relates to a lipid fraction for the support of brain function. The lipid fraction comprises hexanoic acid and/or octanoic acid, eicosapentaenoic acid, and more than 0.4 g [alpha]-linolenic acid per 100 g fatty acids of the lipid fraction for the support of brain function. Optionally, the lipid fraction is administered in combination with non-lipid ingredients, but the lipid fraction preferably contributes to more than 45 en% of the energy of a ready to consume product. Nucleotides may be added to support brain function. Use of fibres is mentioned in order to reduce gastro-intestinal discomfort or to improve postprandial lipid profile. WO 2009/002165 acknowledges that brain function involves various distinct aspects, such as the cognition, social skills, decision making skills and motoric skills. However, even though motoric skills are mentioned as something in which brain function plays a role, there is no specific disclosure of the use of specific combination of components in order to provide a composition that is particularly useful for improving or maintaining the functioning of mammal's limbs.

There is a need for specific products which contribute to improve, maintain or at least reduce a decline in the functioning of a mammal's, in particular a human's, limbs.

The inventors found that a specific combination of components has a positive effect on one or more functions of the limbs in order to improve specific aspects in relation to the functioning of brain functioning, in particular coordination, balance, grip strength, fine motor skills or gross motor skills. Accordingly the invention relates to a combination of a uridine source and an omega-3-polyunsaturated fatty acid having 18-24 carbon atoms for at least one use selected from
- use in the prevention or treatment of a disturbance in coordination of limbs in a mammal.
- use in the prevention or treatment of a disturbance in equilibrium in a mammal.
- in the prevention or treatment of a disturbance in limb strength, in particular forelimb grip strength in a mammal.
- use in the prevention or treatment of a disturbance in a fine-motor skill in a mammal.
- use in the prevention or treatment of a disturbance in a gross-motor skill in a mammal.

Further, the invention relates to the use of a combination of a uridine source and an omega-3-polyunsaturated fatty acid having 18-24 carbon atoms in the manufacture of a medicament or a preparation for at least one of these uses.

Further, the invention, relates to a use, in particular a non-medical use of a uridine source and an omega-3-polyunsaturated fatty acid having 18-24 carbon atoms for improving coordination of limbs in a mammal, for improving sense of equilibrium in a mammal, for improving forelimb grip strength in a mammal, for improving a fine-motor skill in a mammal or for improving a gross-motor skill.

Further, the invention relates to a method of administering a combination suitable for at least one of said uses, comprising administering an effective amount of according to the invention a combination of a uridine source and an omega-3-polyunsaturated fatty acid having 18-24 carbon atoms to a mammal. Such mammal is typically in need of treatment because it suffers from a disturbance in coordination of limbs in a mammal, a disturbance in equilibrium in a mammal, a disturbance in limb strength, in particular forelimb grip strength in a mammal, a disturbance in a fine-motor skill in a mammal, or a disturbance in a gross-motor skill, or because the mammal belongs to a risk group for developing such a disturbance. Such risk groups are in particular mammals suffering from a disorder of which any one or more of said disturbances are known symptoms. In particular, mammals suffering from a disorder mentioned in the present description or claims belong to such risk groups.

It has surprisingly been found that a combination of an omega3-PUFA and a uridine source, said combination optionally comprising one or more further active ingredients, such as vitamin D or butyrate producing fibre, has a positive effect on functioning of a limb. It is considered that the improvement is at least partly due to an improvement in the neurological functioning of the mammal treated with a combination.
Figure 1 shows results for Example 1: the average time on rotarod (in seconds) at different times (in days) after intra-striatal injection with vehicle (Veh) or rotenone (Rot). Four groups of mice are shown as explained in Table 1.
Figure 2 shows results for Example 5, regarding the time spent by mice on a Rotarod in a therapeutic setting.

Motor skills are movements and actions of the muscles. They are categorized in two groups: gross motor skills and fine motor skills. Gross motor skills involve movement of the arms, legs, feet, or entire body. This includes actions such as running, crawling, walking, swimming, and other activities that involve larger muscles. Fine motor skills are the small movements that occur in the hands, wrists, fingers, feet, toes, lips and tongue. They are the smaller actions that occur such as picking up objects between the thumb and finger, using a pencil to write carefully, holding a fork and using it to eat, and other small muscle tasks that occur on a daily basis.

A suitable way to test balancing ability in a mammal (under lab conditions), and thus also to test whether a certain treatment has an effect on a disturbance in equilibrium is a rotarod test, as described in detail in the Examples.

In humans, balance abilities are measured with the Berg Balance Scale (BBS), which is generally considered the gold standard. The BBS is a clinical measure of a person's static and dynamic balance ability. The test takes 15-20 minutes and comprises a set of 14 simple balance related tasks, ranging from standing up from a sitting position, to standing on one foot. The degree of success in achieving each task is given a score of zero (unable) to four (independent), and the final measure is the sum of all of the scores" (http://aahf.info/pdf/Berg Balance Scale.pdf) The BBS is a validated measure to assess balance abilities in neurological disorders such as Parkinson's disease (Qutubuddin et al., Arch Phys Med Rehabil., 2005, Apr; 85(4):789-92 Other tests include the Tinetti Mobility test, which assess also risk of falls in Parkinson disease (Kegelmeyer et al., 2007, Phys Ther. 87(10):1369-78

Fine motor skills can be evaluated in humans using a panel equipped with digital timing sensors, collecting information on speed and accuracy during specific dexterity tasks as described in Smith et al., Neurology, 53(7):1458-1458, 1999.

"Coordination" as used herein generally means "coordination of limbs". Coordination of limbs can be determined by testing the patient's ability to perform rapidly alternating and point-to-point movements correctly. Point-to-point movements are movements performed with the same index finger to bring from one point to another, for instance from the subject's own nose to the examiner's outstretched finger.

Strength can be tested with a calibrated strength tester, designed for testing the strength of a specific limb or part thereof.

"Forelimb grip-strength" can be tested with a calibrated grip strength tester. In a mouse model a grip tester (Panlab, Cornella, Spain) is used by prompting the mouse to grip a trapeze bar with both forelimbs and pulling the mouse by the tail (as proximal to the body as possible) parallel to the orientation of the strain gauge and the trapeze bar. For mean grip strength analyses, a set included five repetitions and relative mean grip strength, i.e. the absolute mean grip strength divided by body weight (grams) was calculated (Leiter et al. 2011) In human, grip strength can be measured with a hand dynamometer.

The term "a" or "an" as used herein is defined as "at least one" unless specified otherwise.

When referring to a noun (*e.g.* a compound, an additive *etc*.) in the singular, the plural is meant to be included.

The term "or" as used herein is to be understood as "and/or" unless specified otherwise.

For the purpose of clarity and a concise description features are described herein as part of the same or separate embodiments, however, it will be appreciated that the scope of the invention may include embodiments having combinations of all or some of the features described.

The term 'fatty acid' is used herein in a way as is common in the prior art. Thus, the fatty acid may be provided as a free fatty acid or salt thereof or in a derivative form - suitable for use in a nutritional of pharmaceutical product - which is derivative form is degradable in the body to release the fatty acid. Suitable derivative forms include esters and ethers, including monoglyceride, diglyceride, triglyceride and phospholipid forms, as known in the art. In particular, good results have been achieved with a triglyceride. When making calculations about amounts, one can assume the same bioavailability as the pure fatty acid and a contribution of fatty acid that is similar to the amount moles of fatty acids in the complete molecule, and correcting for the weight of the complete molecule. Polyunsaturated fatty acids are abbreviated as PUFA's.

The invention is in particular considered useful for treatment of a human, preferably a human of at least 18 years of age, in particular a human of at least 50 years of age, more in particular an elderly human (at least 65 years of age).

When referred to a dosage of a component, the specified dosage is in particular useful for treatment of a human, more in particular for an adult human, unless indicated otherwise.

The combination for use according to the invention is usually administered at least about once a week. Preferably it is at administered at least once per period of three days, more preferably at least once per period of two days, in particular at least once per day, more in particular at least twice per day. In general, the combination is administered up to 10 times, preferably up to 8 times, in particular 5 times per day or less. For embodiments wherein the combination or composition is administered less than once a day, the unit dosages (dosage per serving) of the active ingredients is usually within the range for the daily dosages mentioned elsewhere herein, although the concentration of the active ingredients may be higher.

A combination for use according to any of the invention can be administered as part of a nutritional composition. The nutritional composition may be a fluid drinkable composition, a spoonable composition or a solid composition. Preferably, the nutritional composition is a liquid composition, preferably liquid ready to drink composition. The combination or nutritional composition according to the invention, is preferably administered to the subject in need thereof by oral ingestion. In an alternative embodiment, tube feeding is used.

In particular, in case a nutritional composition for use in accordance with the invention is intended for prophylactic or therapeutic treatment of symptom of a disease, the composition may be a medical food product, *i*.*e*. a food product for use in enhancing, maintaining or restoring health and/or prevent a disease, prescribed by a health care professional like a physician, nurse, or dietician, and destined for and supplied to persons in need thereof.

In yet another embodiment, the combination is administered rectally.

In a preferred embodiment, directed to the combination for a medical use, the mammal is a human suffering from a neurological disorder or a psychiatric disorder or a human at risk of suffering from such disorder, benefits from treatment with a combination for use according to the invention. As used herein 'at risk' means in particular a significantly above average risk.

The neurological disorder is preferably a peripheral neuropathy, an autonomic neuropathy, or an enteric nervous system neuropathy, preferably an autonomic neuropathy, or an enteric nervous system neuropathy.

Preferably, the neurological disorder is a progressive neurodegenerative disease.

Preferably, the neurological disorder is selected to from the group consisting of synucleopathies, diabetic neuropathy, Duchenne's dystrophy, cerebrovascular disease (more particularly stroke), Multiple Sclerosis, pure autonomic failure, and spinal cord injury, preferably selected to from the group consisting of cerebrovascular disease (more particularly stroke), Multiple Sclerosis, pure autonomic failure, spinal cord injury, and Parkinson's disease.

In particular, good results have been achieved with a combination of the invention using a mouse model for Parkinson's disease. In a particularly preferred embodiment, the combination of the invention is useful to improve coordination or balance in a Parkinson's patient.

In a specific embodiment, the combination is for use of a subject suffering from cerebral palsy.

Preferably, the psychiatric disorder is a pervasive development disorder, preferably an austistic spectrum disorder.

Preferably, the psychiatric disorder is depression, in particular a depressive mood disorder.

In a further preferred embodiment, the combination of the invention is for use in the treatment of a human suffering from dementia, in particular Alzheimer Disease. Based on tests with Alzheimer patients, the inventors consider that dementia patients, in particular may benefit from treatment with a combination of the invention with respect to a fine motor skill or limb strength, such as handgrip strength.

Preferably, the omega-3 polyunsaturated fatty acids is selected from the group consisting of docosahexaenoic acid (DHA), docosapentaenoic acid (DPA), and eicosapentaenoic acid (EPA).

The PUFA, in particular DHA, EPA and/or DPA, is preferably provided as triglyceride, diglyceride, monoglyceride, free fatty acid or salt or ester, phospholipid, lysophospholipid, glycerol ether, lipoprotein, ceramide, glycolipid or combinations thereof.

More preferably, the combination for use according to the invention comprises at least the omega3-PUFA, in particular DHA, in triglyceride form.

The present combination for use according to the invention preferably comprises 1-40 wt.% DHA based on total fatty acids, preferably 3-36 wt.% DHA based on total fatty acids, more preferably 10-30 wt.% DHA based on total fatty acids.

The present combination for use according to the invention preferably comprises 0.5-20 wt.% EPA based on total fatty acids, preferably 2-10 wt.% EPA based on total fatty acids, more preferably 5-10wt.% EPA based on total fatty acids.

The proportion of DHA+EPA of the total fatty acids present in the combination for use according to the invention usually is 55 wt. % or less, preferably 5-50 wt.%, more preferably 10-45 wt.%, most preferably 15-40 wt.%.

The daily dosage of polyunsaturated fatty acid having 18-24 carbon atoms administered via the combination usually is in the range of 0.4-15 grams, preferably in the range of 0.6-12 g, more preferably in the range of 1.0-10 g, in particular in the range of 1.5-5 g.

In particular, good results have been achieved with DHA, especially in combination with butyrate producing fibre. If present in the combination for use according to the invention DHA is preferably present in sufficient amount for the administration of DHA in an amount of 300 to 4000 mg per day, more preferably 500-2500 mg per day.

If EPA and/or DHA are present, the total daily dosage of DHA plus EPA taken together generally is in in the range of 300-7500 mg/day, preferably in the range of 500-6500 mg/day, more preferably in the range of 1000-5000 mg/day, in particular 1000 - 4000 mg/day, more in particular 1500-3000 mg/day.

In particular, good results have been achieved with a combination comprising both EPA and DHA. If both are present, the weight to weight ratio EPA:DHA usually is in the range of 1:99 to 99:1, preferably in the range of 1:10 - 5:1, in particular in the range of about 1:5 - 1:2, more in particular about 1:3 - 1:2.

In terms of DHA content in a nutritional composition that is administered in accordance with the invention, the DHA content preferably is 5 g/100 gram nutritional composition or less, in particular 1-4 g/100 g nutritional composition.

Preferably, a combination for use according to the invention comprises fish oil providing the omega-3-PUFA. Another particularly suitable source for the omega-3-PUFA is algae oil.

The present combination for use according to the invention preferably comprises less than 5 wt.% arachidonic acid based on total fatty acids, more preferably below 2.5 wt.%, e.g. down to 0.5 wt%.

Preferably the weight ratio DHA/AA in the present combination for use according to the invention is at least 5, preferably at least 10, more preferably at least 15, preferably up to e.g. 30 or even up to 60. Evidently, the ratio may be higher and approximate infinity if AA is absent (non-detectible).

The ratio omega-6/omega-3 fatty acids in the present combination for use according to the invention is preferably below 0.5, more preferably below 0.2, e.g. down to 0.05 or to 0.01. In particular, the ratio omega-6/omega-3 fatty acids (C 20 and higher) in the present combination for use according to the invention is preferably below 0.3, more preferably below 0.15, e.g. down to 0.06 or to 0.03.

The uridine source typically is selected from uracil, (deoxy)uridine, nucleotides of (deoxy)urdine, cytosine, (deoxy)cytidine, nucleotides of (deoxy)cytidine and derivatives thereof. Dietary cytidine, which is capable of being converted into uridine in humans, is regarded as a source of uridine in the context of the present invention.

Regarding the derivatives, in particular an amino group attached to the heterocyclic six membered ring may be derivatised or one or more of the hydroxyl groups of the ribose or deoxyribose of the nucleoside or nucleotide may derivatised, preferably esterified (acylated), for instance with a C1-C24 carboxylic acid. Preferred acylated forms of the uridine source are those wherein the (deoxy)ribose has been acylated with acetic acid, n-caproic acid, caprylic acid, or n-capric acid, because these increase the bioavailability of the uridine source. Methods for reacting these medium chain fatty acids to uridines, for example to the 5' position of the uridine are known in the art per se for other fatty acids and comprise conventional acylation methods. In a further embodiment the uridine source is acylated with a PUFA, for instance an omega-3 PUFA. Thus, in a specific embodiment, the combination for use of the invention comprises the PUFA and the uridine source combined in a single molecule, which may be hydrolysed *in vivo.* Esters of a uridine/cytidine derivatives and methods for making those are generally known in the art. Such derivatives have amongst others been described in EP 1,390,378 and in US 5,470,838.

Preferred uridine sources in a combination for use according to the invention the uridine source are selected from the group of uracyl, uridine, deoxyuridine, derivatised uridine, derivatised deoxy uridine, cytosine, (deoxy)cytidine and derivatised cytidines; more preferably a uridine source is selected from the group of uridine, UMP, UDP, UTP, CMP, CDP, CTP dUMP, dUDP, dUTP, dCMP, dCDP and dCTP. Optionally one or more hydroxyl moieties of the (deoxy)ribose of said nucleotide or nucleoside is acylated.

Particularly preferred is a uridine source selected from the group of uridine UMP, UDP, UTP, dUMP, dUDP, dUTP, wherein optionally the (deoxy)ribose of said nucleotide or nucleoside is acylated.

In a highly preferred embodiment, the present combination for use according to the invention comprises uridine and/or uridine phosphate.

Preferably at least 50 wt.% of the uridine source in the combination for use according to the invention is provided by UMP, more preferably at least 75 wt.%, most preferably at least 95 wt.%.

The present combination for use according to the invention is preferably administered (or is in a format) to provide uridine (the cumulative amount of uridine, deoxyuridine, uridine phosphates, uracil and acylated uridine derivatives) in an amount of 0.08-3 g per day, preferably 0.1-2 g per day, more preferably 0.2-1 g per day.

The present combination for use according to the invention preferably comprises uridine in an amount of 0.08-3 g UMP per 100 ml of a liquid product, preferably 0.1-2 g UMP per 100 ml of a liquid product, more preferably 0.2-1 g per 100 ml liquid product.

Preferably 1-37.5 mg UMP per kilogram body weight is administered per day.

The required dosages of the equivalents on a weight base can be calculated from the dose for UMP by taking equimolar amounts using the molecular weight of the equivalent and of UMP, the latter being (about) 324 Dalton. Accordingly a daily dosage of the uridine source in the range of 3-116 µmol per kg body weight per day is preferred. In a particularly preferred embodiment the daily dosage of uridine source is 10-50 µmol per kg body weight per day . If desired a lower amount or higher amount, e.g. from about 1 to about 150 µmol/kg body weight can be administered.

In a preferred embodiment, the combination for use according to the invention comprises dietary fibre.

The fibres may be selected from soluble fibres and insoluble fibres.

The fibres are generally composed of a plurality of carbohydrate units. The fibres may be short-chain indigestible carbohydrates or long-chain indigestible carbohydrates. Dependent on the type of fibre, national food regulations may have different definitions for what constitute short chain or long chain. As used herein, short chain fibres generally have a polymerization degree of less than 20, in particular of 2-12 less, more in particular 3-9; long chain fibres have a polymerization degree at least one higher than specified for short chain fibres, so 10 or more, 13 or more or 20 or more.

The inventors surprisingly found that the presence of a specific group of fibres in combination with the omega-3-PUFA and the uridine source has a positive effect on specific body functions wherein the limbs play a role. As illustrated by the Examples, mice treated with a diet containing butyrate producing fibre performed better in a rotarod test than mice treated with a diet without such fibre, indicating improved coordination and/or body balance (equilibrium). From the Examples, the inventors realise that in particular Parkinson patients may benefit from the butyrate producing fibre in combination with the uridine source, omega3-PUFA and optionally one or more other components, such as vitamin D. Without being bound by theory, the inventors contemplate that these fibres produce butyrate which is absorbed by the body and provides a neuroprotective effect relevant to improve coordination or body balance. The inventors further consider that butyrate producing fibre is positive for improving for limp grip strength or a fine motor skill.

Butyrate producing fibres are indigestible carbohydrates that have butyrate as a breakdown product when fermented by colonic flora. The term 'butyrate producing fibre' is used herein in particular for fibre that is capable of producing at least 0.5 mmol or more, preferably at least 0.75 mmol or more, more preferably at least 1.0 mmol or more butyrate / gram fibre after 24 hours of *in vitro* fermentation. In practice, the maximum amount of butyrate producible under these conditions is usually 5 mmol or less, in particular 3.5 mmol or less, more in particular 2.5 mmol or less butyrate / gram fibre after 24 hours of *in vitro* fermentation.

The producible amount of butyrate is determinable using a semi-dynamic colon model (e.g. using a TIM artificial gut system). In an alternative model, fresh faecal samples are collected from healthy adults (without gastrointestinal problems; no use of antibiotics for a last 2 weeks prior to sample taking). Faecal samples are divided in smaller portions and mixed with glycerol (10%) in an anaerobic cabinet and stored at -80°C. In each experiment the faecal samples from all donors are pooled at equal concentrations and mixed together in an anaerobic cabinet, to avoid subject-dependent variation in the adult microbiota as much as possible. The samples are inoculated with a fibre under fermentation conditions and fermentation is allowed to take place. Butyrate production is determined after 24 hrs using GC. In particular
- the model makes use of samples from four healthy adults (e.g., three male donors and one female donor) in the age of 19-35 years;
- the fibre is inoculated in a faeces suspension at a content of 200 mg/ 6 ml faeces suspension
- the faeces suspension is made by mixing aeces with a fermentation medium as 1:5 v/v;
- the fermentation medium: buffered peptone water 3.0 g/l, Yeast Extract 2.5 g/l, Tryptone 3.0 g/l, L-Cysteine-HCl 0.4 g/l, Bile salts 0.05 g/l, K2HPO4.3H2O 2.6 g/l, NaHCO3 0.2 g/l, NaCl 4.5 g/l, MgSO4.7H2O 0.5 g/l, CaCl2. 2H2O 0.3 g/l, FeSO4.7H2O 0.005 g/l. Ingredients can be added one by one in 800 ml water, pH is adjusted to 6.3±0.1 with K2HPO4 or NaHCO3 and volume is filled up to 1 liter. Medium is sterilized for 15 minutes at 121°C
- The fermentation temperature is 37 °C

The total dosage of butyrate producing fibre in accordance with the invention preferably is 1to 15 g per dosage, more preferably of 2 to 10 g per dosage, in particular 3-8 g per dosage. The total butyrate producing fibre content, based on total fibre content in a composition for use in accordance with the invention is up to 100 wt %, in particular 99 wt. % or less, more in particular 95 wt. % or less. The total butyrate producing fibre content, based on total fibre content preferably is at least 50 wt. %, based on total fibre content, preferably at least 70 wt. %, more preferably at least 80 wt. %, in particular 90 wt. % or more.

Preferably the combination or nutritional composition for use according to the invention comprises fibre in an amount sufficient to produce 0.3-5 mmol, more preferably 0.5-3.5, in particular 0.7-3, more in particular 1.0-2.5 mmol butyrate per gram fibre, under the using a semi-dynamic colon model, described above.

Preferred soluble butyrate producing fibres include fructooligosaccharides (FOS), galactooligosaccharides (GOS), bran and dextrins (*e.g.* Nutriose®). These are well soluble and are a suitable substrate for colonic flora to produce butyrate, when fermented. Preferred brans are oat bran, rice bran and wheat bran. In a particularly preferred embodiment, several butyrate producing fibres are used, such as at FOS and a dextrin plus optionally bran and/or GOS; or FOS and bran plus optionally dextrin and/or GOS. Good results have been achieved with a combination of short chain FOS, long chain FOS, oat bran, GOS and dextrin. In an other preferred embodiment, the combination is part of a nutritional composition that comprises one or more butyrate producing fibres but that is essentially free of GOS. Such composition preferably is a milk-free composition.

Preferred insoluble butyrate producing fibres include resistant starch, such as high-amylose starch or retrograded or RS3 starch. Resistant starch is suitable to provide a particularly high butyrate production per g of resistant starch.

Resistant starch is defined to be as those starches which remain intact after digestion during 2 hours in the system of Englyst et al Am J Clin Nutr 1999, 69, 448-454. Commercially available resistant starches are Actistar and Novelose 330.

Preferred resistant starches are resistant starches from rice or corn.

In a specific embodiment, the resistant starch comprises more than 50wt% linear polymers of alpha 1,4 glucans which have a degree of polymerization between 10 and 35. Suitable sources of such resistant starches are beans, peas, heat-treated potatoes and heat-treated cereals. Simultaneous presence in the colon of resistant starch and beta glucans, in combination with a xylan will support of growth of the right type of butyrate generating bacteria species.

.Preferably a combination of the invention, in particular a nutritional composition to be used according to the invention further comprises a vitamin, preferably at least one vitamin selected from the group of, vitamin E, vitamin C, vitamin D, vitamin B12, vitamin B6 and folic acid.

Advantageously, vitamin B6, vitamin B12 and folate are included. It has been found in particular that a nutritional composition comprising these three vitamins has a beneficial effect on fine motor skills or in improving strength, such as handgrip. In particular, Alzheimer Disease patients benefit from the presence of these vitamins in a combination of the invention, although their presence may also benefit other subjects, in particular other subjects suffering from a neurological or psychiatric disease with respect to a fine motor skill, strength or other use of the invention.

A combination for use in accordance with the invention preferably comprises 50 to 1000 pg folic acid, more preferably 150 to 750 pg, most preferably 200 to 500 pg folic acid, per 100 ml liquid product.

The daily folic acid dosage in accordance with the invention preferably is 50-1000 pg folic acid per day, more preferably 150-750 pg, most preferably 200 - 500 pg folic acid per day.

The present nutritional composition preferably comprises 0.5 to 15 pg vitamin B12, more preferably 1 to 10 pg, most preferably 1.5 to 5 µg vitamin B12, per 100 ml liquid product.

The daily vitamin B12 dosage in accordance with the invention preferably is 0.5-15 pg vitamin B12 per day, more preferably 1-10 pg, most preferably 1.5-5 pg vitamin B12 per day.

Advantageously, the nutritional composition in a use according to the invention comprises 0.5 to 3 mg, preferably 0.5-2 mg vitamin B6, per 100 ml liquid product.

Preferably, each of vitamin B6, vitamin B12 and folate are included.

In a highly preferred embodiment, the combination for use in accordance with the invention comprises vitamin D. An advantage of vitamin D is a reduced balance impairment and reduced risks of falls. In particular, the inventors realized that Parkinson patients benefit from vitamin D with respect to these effects, although other subjects - in particular humans suffering from another neurological disorder or a psychiatric disorders - may also have a benefit from vitamin D with respect to a use of the invention.

Further, the inventors found a positive effect of vitamin D in a combination of the invention on grip strength. In particular, the presence of vitamin D in a combination of the invention is considered beneficial to limb strength of an elderly person, a Parkinson patient or a dementia patient, e.g. an Alzheimer patient. It is in particular surprising the vitamin D has an effect in an elderly subject, a dementia patient or a Parkinson patient. Earlier scientific papers reported that vitamin D did not have an effect on strength in elderly people (Smedshaug et al, Food & Nutrition, VOl 51, No 2 (2007) p 74-78 ...

Good results have been obtained with vitamin D3 (cholecalciferol, calcifediol, calcitriol).

If present, the concentration of vitamin D, preferably of vitamin D3, in a nutritional composition for use in accordance with the invention usually is in the range of 5-110 µg/100, in particular in the range of 6-85 µg/100g preferably in the range of 10-50µg/100g, more preferably 15-45 µg/100g.

The combination for use according to the invention usually provides vitamin D to the mammal, in particular human, that is treated in a in a daily dosage of up to about 50 µg, preferably 25 -40 µg. For a liquid product, the vitamin D3 content preferably is 5-85 µg per unit dosage. The unit dosage of a liquid product preferably has a volume of 50-250 ml, in particular 100-150 ml.

In a specific embodiment, the nutritional composition for use according to the invention comprises one or more trace elements, in particular selenium.

Preferably, the nutritional composition comprises at least 2, 3, 4, 5, or 6 components selected from the group o of phospholipids, vitamin E, vitamin C, selenium, vitamin B12, vitamin B6 and folic acid, even more preferably comprises phospholipids, vitamin E, vitamin C, selenium, vitamin B12, vitamin B6 and folic acid.

In a specific embodiment, the nutritional composition contains choline. Preferably the present nutritional composition contains choline and/or phosphatidylcholine. Preferably the nutritional composition is administered (or is in a format) for providing more than 50 mg choline per day, preferably 80-2000 mg choline per day, more preferably 120-1000 mg choline per day, most preferably 150-600 mg choline per day.

Preferably the present nutritional composition contains 50 mg to 3 gram choline per 100 ml of a liquid formula, preferably 200 mg - 1000 mg choline/100 ml.

The present nutritional composition may advantageously contain phospholipids. As used herein, the term phospholipid includes lyso-phospholipids, de-acylated phospholipids and glycerophospholipids. Preferably a phospholipid is provided in the form of lecithin. The present nutritional composition is preferably a liquid composition, wherein lecithin is provided in an amount of 0.01 and 1 gram lecithin per 100 ml, more preferably between 0.05 and 0.5 gram lecithin per 100 ml. The presence of lecithin is in particular preferred in case a human suffering from progressive neurodegenerative disease, such as Parkinson's disease is treated.

Further, preferred phospho¬lipids are selected from the group of phosphatidylcholine(PC), phosphatidylethanolamine (PE), phosphatidylserine (PS), phosphatidic acid (phosphatidate), phosphoinositides (such as phosphatidylinositol (PI), phosphatidylinositol phosphate, phosphatidyl¬inositol bisphosphate, phosphatidylinositol triphosphate) and sphingomyelin. In particular an elderly subject or a dementia patient, such as an Alzheimer patient may benefit from any one or more, preferably two or more of the these phospholipids.

The nutritional composition usually further comprises a digestible carbohydrate fraction (e.g. sugars, digestible starch, which may be partially hydrolysed) and/or a proteinaceous matter fraction (peptides, proteins, amino acids, including derivatives thereof that still contribute to energetic value of the composition, e.g. hydrolysable esters). Further, the composition may comprise a lipid fraction comprising the PUFA and one or more other lipids.

In an advantageous embodiment, the lipids account for less than 50 en% of the nutritional composition, in particular 46 wt. % or less, more in particular 43 wt. % or less, or 40 wt. % or less. Usually, the lipids (including he omega-3 PUFA) provide at least 1 en %, preferably at least 5 en %, in particular at least 10 en % of the nutritional composition. Proteinaceous matter and/or digestible carbohydrates (non-lipid component having a caloric value) typically provide the balance of the energetic value. For energetic value the Atwater factors are used: for digestible carbohydrates (4 kcal/g), proteins (4 kcal/g) and lipids (9 kcal/g), and zero for others, such as the organic acids and nutritional fiber, minerals and vitamins.

In a specific embodiment, the nutritional composition for use in accordance with the invention comprises
i-) at least one component selected from the group of uridine, acylated uridine, uridine monophosphate and acylated uridine monophosphate.
ii) DHA and EPA
iii) a vitamin B selected from the group of vitamin B6, vitamin B9 and vitamin B12
iv) a phospholipid
v) an antioxidant selected from the group of vitamin C, vitamin E and selenium
vi) a choline
vii) a protein.

Such nutritional composition is particularly useful for treatment of an Alzheimer patient in accordance with the invention, also if such product is essentially free of butyrate producing fibres and/of vitamin D.

The composition of this specific embodiment preferably comprises phosphatidylcholine, and the weight to weight ratio phosphatidylcholine to choline preferablt is more than 0.26.

The total content of uridine source in this specific embodiment preferably is in the range of 5-30 mg per gram dry weight, more preferably in the range of 8-20 mg per gram dry weight, in particular in the range of 10-18 mg per gram dry weight.

The nutritional composition of this specific embodiment preferably comprises 5-10 mg EPA per gram dry weight and 2.5-20 mg DHA per gram dry weight.

The nutritional composition of this specific embodiment preferably has a protein content is less than 400 mg per gram dry matter, preferably 100-340 mg protein per gram dry matter. Preferably whey protein is present. Further advantageous proteins in particular include fish proteins (in particular cod protein), egg-protein and vegetable proteins. The total content of non-dairy protein preferably is more than 21 wt. % ,more preferably 22-80 wt. %, in particular 25-40 wt. %.

The nutritional composition of this specific embodiment preferably comprises 0.15-0.5 g digestible carbohydrate per gram dry weight, in particular 0.20-0.40 g digestible carbohydrate per gram dry weight.

The nutritional composition of this specific embodiment preferably comprises 0.15-0.3 g lipids per gram dry weight.

The energy density of a composition according to this specific embodiment typically is less than 13 kcal per gram dry matter, preferably of 3-9.3 kcal per gram dry matter, more preferably of 4.0-7.0 kcal per gram dry matter.

For ease of administration to a patient, in particular an Alzheimer patient, the nutritional composition of this specific embodiment is preferably administered as a fluid. It may be packaged as a ready-to-use fluid or be an instant product (powder) to be reconstituted prior to use. The fluid typically has a dry matter content of 15-30 g per 100 ml, in particular of 16-24 g per 100 ml, more in particular of 17-22 g per 100 ml. The osmolarity of the fluid composition typically is 120 to 450 mEq/l.

A combination for use according to the invention or used in accordance with the invention may in particular be a combination or use mentioned in the following items The invention further relates to the following items:
1. A combination of a uridine source and an omega-3-polyunsaturated fatty acid having 18-24 carbon atoms for use in the prevention or treatment of a disturbance in coordination of limbs in a mammal.
2. A combination of a uridine source and an omega-3-polyunsaturated fatty acid having 18-24 carbon atoms for use in the prevention or treatment of a disturbance in equilibrium in a mammal.
3. A combination of a uridine source and an omega-3-polyunsaturated fatty acid having 18-24 carbon atoms for use in the prevention or treatment of a disturbance in limb strength, in particular forelimb grip strength in a mammal.
4. A combination of a uridine source and an omega-3-polyunsaturated fatty acid having 18-24 carbon atoms for use in the prevention or treatment of a disturbance in a fine-motor skill in a mammal.
5. A combination of a uridine source and an omega-3-polyunsaturated fatty acid having 18-24 carbon atoms for use in the prevention or treatment of a disturbance in a gross-motor skill in a mammal.
6. A combination for use according to any of the preceding items, wherein the mammal suffers from a neurological disorder or a psychiatric disorder.
7. A combination for use according to item 6, wherein the neurological disorder is a peripheral neuropathy, an autonomic neuropathy, or an enteric nervous system neuropathy, preferably an autonomic neuropathy, or an enteric nervous system neuropathy.
8. A combination for use according to item 6 or 7, wherein the neurological disorder is selected to from the group consisting of synucleopathies, diabetic neuropathy, Duchenne's dystrophy, cerebrovascular disease, Multiple Sclerosis, pure autonomic failure, and spinal cord injury, preferably selected to from the group consisting of cerebrovascular disease, Multiple Sclerosis, pure autonomic failure, spinal cord injury, and Parkinson's disease.
9. A combination for use according to item 8, wherein the neurological disorder is spinal cord injury.
10. A combination for use according to item 8, wherein the neurological disorder is Parkinson's disease.
11. A combination for use according to item 6, wherein the neurological disorder is stroke.
12. A combination for use according to item 6, wherein the mammal suffers from a neurological disorder and the neurological disorder is dementia, preferably Alzheimer Disease.
13. A combination for use according to item 6, wherein the psychiatric disorder is pervasive development disorder, preferably austistic spectrum disorder, or the psychiatric disorder is depression, in particular a depressive mood disorder.
14. A combination for use according to any of items 1 to 13, wherein the omega-3 polyunsaturated fatty acid is selected from the group consisting of docosahexaenoic acid (DHA), docosapentaenoic acid (DPA), and eicosapentaenoic acid (EPA).
15. A combination for use according to item 14, comprising DHA.
16. A combination for use according to item 15, wherein the daily dosage of DHA plus EPA administered via the combination is in the range of 400 mg to 7500 mg, preferably in the range of 500 mg to 5000 mg, in particular in the range of 1000 mg to 3000 mg.
17. A combination for use according to any of the preceding items, wherein the uridine source comprises a nucleotide selected from the group of UMP, UDP, UTP, CMP, CDP, CTP dUMP, dUDP, dUTP, dCMP, dCDP and dCTP, wherein optionally one or more hydroxyl moieties of the (deoxy)ribose of said nucleotide are acylated.
18. A combination for use according to item 17, wherein the uridine source is selected from the group consisting of non-acylated UMP and acylated UMP's.
19. A combination for use according to any of the preceding items, wherein the uridine source comprises a nucleoside selected from the group of uridine and deoxyuridine, wherein optionally one or more hydroxyl moieties of the (deoxy)ribose of said nucleoside are acylated.
20. A combination for use according to any of the preceding items, wherein the daily dosage of the uridine source is in the range of 1-150 µmol per kg body weight per day, preferably in the range of 3-116 µmol per kg body weight per day.
21. A combination for use according to any of the preceding items wherein the combination comprises a dietary fibre.
22. A combination for use according to item 21, wherein the combination comprises a dietary fibre selected from the group of butyrate producing fibres.
23. A combination for use according to item 22, wherein at least one butyrate producing fibre is present selected from the group of fructooligosaccharides (FOS), galactooligosaccharides (GOS) and dextrins (e.g. Nutriose®).
24. A combination for use according to any of the items 21-23, wherein the combination is part of a nutritional composition that is essentially free of galactooligosaccharides.
25. A combination for use according to item 22, 23 or 24, wherein the butyrate producing fibre is to be administered in a dosage of 3-8 grams.
26. A combination for use according to any of the preceding items, wherein the combination is to be administered as part of a nutritional composition, further comprising a phospholipid.
27. A combination for use according to any of the preceding items , wherein the combination is to be administered as part of a nutritional composition, further comprising at least one component selected from the group of vitamin E, vitamin C and selenium.
28. A combination for use according to any of the preceding items, wherein the combination comprises at least one vitamin selected from the group of vitamin B12, vitamin B6 and folic acid, preferably comprising vitamin B12, vitamin B6 and folic acid.
29. A combination for use according to item 28, wherein the combination is part of a nutritional composition, the nutritional composition comprising
   i-) at least one component selected from the group of uridine, acylated uridine, uridine monophosphate and acylated uridine monophosphate.
   ii) DHA and EPA
   iii) a vitamin B selected from the group of vitamin B6, vitamin B9 and vitamin B12
   iv) a phospholipid
   v) an antioxidant selected from the group of vitamin C, vitamin E and selenium
   vi) a choline
   vii) a protein.
30. A combination for use according to any of the preceding items, wherein the combination further comprises vitamin D, preferably vitamin D3.
31. A combination for use according to item 30, wherein vitamin D, preferably vitamin D3, is to be administered in a daily dosage of 5-50 µg, in particular 25-40 pg.
32. A combination for use according any of the preceding items, wherein the combination is to be administered into the gastro intestinal tract.
33. A combination for use according to item 31, wherein the combination is to be administered orally.
34. A combination for use according to any of the preceding items, wherein the combination is part of a nutritional composition, further comprising at least non-lipid components having a caloric value selected from the group of proteinaceous matter and digestible carbohydrates, wherein the total caloric value of said non-lipid components is at least 50 en %, preferably 55 en% or more, in particular 60 en % or more.
35. A combination for use according to any of the preceding items, wherein the mammal is a human, preferably a human of at least 18 years of age, in particular an elderly human.
36. A combination for use according to any of the preceding items, wherein the mammal is a subject suffering from cerebral palsy.
37. Non-medical use of a uridine source and an omega-3-polyunsaturated fatty acid having 18-24 carbon atoms for improving coordination of limbs in a mammal, for improving sense of equilibrium in a mammal, for improving forelimb grip strength in a mammal, for improving a fine-motor skill in a mammal or for improving a gross-motor skill.
38. Non-medical use according to items 31-34, wherein the combination is as defined in any of the items 13-35.

The invention is now illustrated by the following examples.

### Example 1

In mice, Parkinson's disease (PD), was induced by intra-striatal injection of 2.7 µg of rotenone by stereotaxic surgery into the brain. Epidemiological studies had previously shown that exposure to this pesticide induced Parkinson's disease (PD). The rotarod treadmill consists of a plastic rod, with a non-slippery surface, above the base (trip plate). Mice were placed on an accelerating rod with speeds starting with 2 rpm and gradually increasing to 20 rpm. The rodent's ability to remain on the rotating rod (time to first fall) was recorded for a maximum of 5 minutes. Before baseline, mice were trained with the rotarod apparatus to rule out learning effects throughout the experiment.

Four experimental groups of 10 mice each were used which were either injected with rotenone or vehicle and which received control or active diet (=UMP+ fish oil) according to Table 1. The specific composition of the control and active diets are presented in Table 2. Dietary intervention was started one week before surgery and was continued for six weeks.

**Table 1 Experimental groups**

| Group | Intra-striatal injection | Diet |
|---|---|---|
| Veh/CF | Vehicle | Control |
| Veh/AF | Vehicle | UMP + fish oil |
| Rot/CF | Rotenone | Control |
| Rot/AF | Rotenone | UMP + fish oil |

| Table 2 Composition of the diets | | |
|---|---|---|
| | | |
| | | |
| animal food based on AIN -93 M Reeves, et al. (1993) | **g/100 g diet** | **g/100 g diet** |
| | **Control** | **Fish+UMP** |
| Corn starch | 31.10 | 29.30 |
| Casein (>85% protein) | 14.00 | 14.00 |
| corn dextrine | 15.50 | 15.50 |
| Sucrose | 10.00 | 10.00 |
| Dextrose | 10.00 | 10.00 |
| | | |
| Fibers | 5.00 | 5.00 |
| | | |
| Minerals mix (AIN-93M-MX) (*) | 3.50 | 3.50 |
| Vitamins mix (AIN-93-VX) (*) | 1.00 | 1.00 |
| | | |
| **Fat** | 4.77 | 4.77 |
| saturated fat | 1.3 | 1.2 |
| Monounsaturated fatty acids (MUFA) | 1.100 | 1.100 |
| PUFA | 2.300 | 2.200 |
| EPA (C-20:5w3) | 0.000 | 0.300 |
| DHA (C-22:6w3) | | 0.700 |
| | 0.000 | |
| | | |
| | | |

| **Additions:** | | |
|---|---|---|
| L-cystine | 0.180 | 0.180 |
| Choline bitartrate (41.1% choline) | 0.250 | 0.250 |
| Tert-butylhydroquinone | 0.0008 | 0.0008 |
| | | |
| | | |
| UMP disodium (24%H2O) | 0 | 1.0 |
| | | |
| Total | 100.0 | 100.0 |
| Energy | 344.6 | 345.5 |

| | | |
|---|---|---|
| (*) See Reeves 1993 - J Nutr; Nov; 123(11); 1939-51. | | |

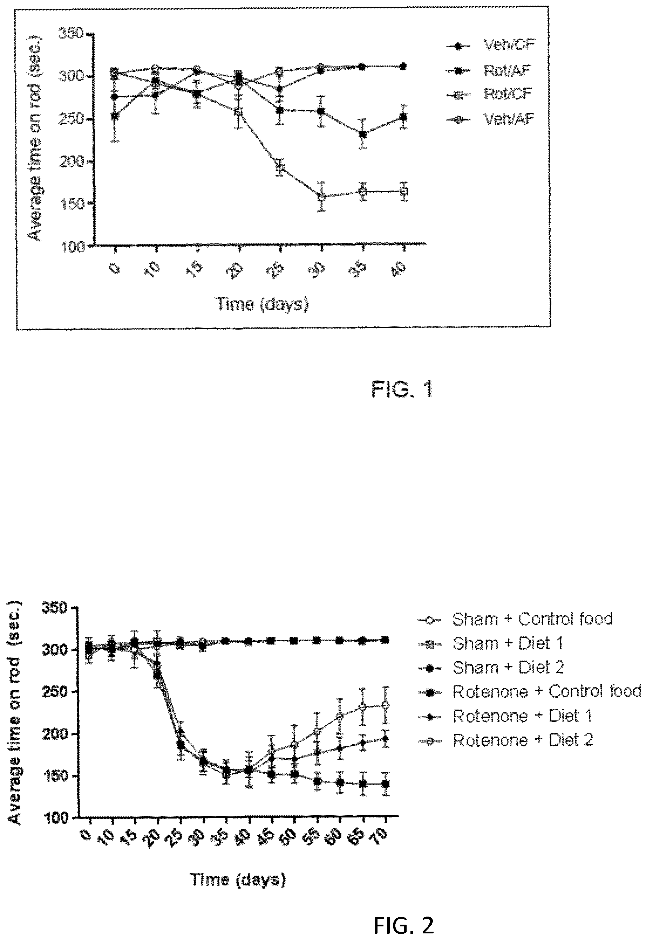

The intra-striatal injection with rotenone resulted in the well-known motor symptoms of PD, shown by the impaired ability of the mice to remain on a moving rotarod. The ability to remain on the rod was much better (more than 50 % higher average time on the rod) in mice treated with a combination of the invention (Ror/AF) after treatment, as illustrated by Figure 1.

### Example 2:

Ready to use liquid product providing per 100 mL:
Energy: 50-120 kcal
Protein: 0-10 g
Lipids: 1-5 g
Digestible carbohydrates: 4-30 g
and which comprises:
   DHA+EPA+DPA= 1000-2000 mg
   Uridine monophosphate (disodium salt) or uridine 0.5 g

### Example 3

A preferred nutritional composition according to the invention comprises, per daily dose or per 100 ml composition:
100 - 500 mg, preferably 200-400 mg EPA,
900 - 1500 mg, preferably 950-1300 mg DHA
50 - 600 mg, preferably 60-200 mg phospholipids,
200 - 600 mg, preferably 300-500 mg choline,
400 - 800 mg, preferably 500-700 mg UMP or an equivalent molar amount of uridine,
20 - 60 mg, preferably 30-50 mg vitamin E (alpha-TE),
60 - 100 mg, preferably 60-90 mg vitamin C,
40 - 80 pg, preferably 45-65 pg selenium,
1 - 5 pg, preferably 2-4 µg vitamin B12,
0.5 - 3 mg, preferably 0.5-2 mg vitamin B6, and
200 - 600 pg, preferably 300-500 pg folic acid.

### Example 4

Liquid ready to use product as in Example 2 or 3 for diabetic patient suffering from neuropathy, in which the carbohydrate fraction is composed of (per 100 mL):
1.5 g galactose
3 g palatinose
1 g slowly digestible starch
0.2 g fructose
3 g maltodextrins
2 g glucose
1.05 g isomalto oligosaccharides

### Example 5

The effects of a diet comprising a pyrimidine derivative (UMP) and a PUFA source (DHA) in a therapeutic setting were studied, wherein treatment with a diet according to the invention was initiated 4 weeks after injection with 5.4 µg rotenone or a vehicle and continued for seven weeks. Six experimental groups of male C57Bl/6J mice were used which were either treated with vehicle (Sham) or rotenone and which either received a control diet or a diet according to the invention (diet 1 or diet 2), as indicated in the following table:

**Table 3:. Experimental groups**

| Group | Intra-striatal injection | diet |
|---|---|---|
| Sham+Control Food (control) | Sham | Control |
| Sham+Diet 1 (control) | Sham | Uridine source+ DHA |
| Sham+Diet 2 (control) | Sham | Uridine source + DHA + butyrate producing fibres |
| Rotenone+Control Food | Rotenone | Control |
| Rotenone+Diet 1 | Rotenone | Uridine source + DHA |
| Rotenone+Diet 2) | Rotenone | Uridine source + DHA + butyrate producing fibres |

The compositions of the control and active diets are presented in the following Table.

| Table 4: Composition of the diets | | | | |
|---|---|---|---|---|
| animal food based on AIN -93 M Reeves, et al. (1993) | | g / 100 g diet | g / 100 g diet | g/100 g diet |
| | | Control | Diet 1: Fish oil+UMP | Diet 2: Fish oil+Uridine+buty-rate producing fibers |
| Corn starch | | 31.10 | 29.30 | 31.97 |
| Casein (>85% protein) | | 14.00 | 14.00 | 14.00 |
| corn dextrine | | 15.50 | 15.50 | 14.20 |
| Sucrose | | 10.00 | 10.00 | 10.00 |
| Dextrose | | 10.00 | 10.00 | 10.00 |
| | | | | |
| Fibers (cellulose) | | 5.00 | 5.00 | 0 |
| Butyrate producing fibers | | 0 | 0 | 7.27¹ |
| Minerals mix (AIN-93M-MX) (*) | | 3.50 | 3.50 | 3.50 |
| Vitamins mix (AIN-93-VX) (*) | | 1.00 | 1.00 | 1.00 |
| | | | | |
| Fat | | 4.77 | 4.77 | 4.77 |
| saturated fat | | 1.3 | 1.2 | 1.2 |
| Monounsaturated fatty acids (MUFA) | | 1.1 | 1.1 | 1.1 |
| PUFA | | 2.3 | 2.2 | 2.2 |
| EPA (C-20:5w3) | | 0.0 | 0.3 | 0.5 |
| DHA (C-22:6w3) | | 0.0 | 0.7 | 0.7 |
| | | | | |
| | | | | |
| Additions: | | | | |
| L-cystine | | 0.180 | 0.180 | 0.180 |
| Choline bitartrate (41.1% choline) | | 0.250 | 0.250 | |
| | | | | 0.922 |
| | | | | |
| | | | | |
| Tert-butylhydroquinone | | 0.0008 | 0.0008 | 0.0008 |
| Additional vitamins² | | | | 0.688 |
| Soy lecithin | | | | 0.755 |
| UMP disodium (24%H2O) | | 0 | 1.0 | |
| Uridine (pure) | | | | 0.511 |
| Total | | 100.0 | 100.0 | 100.0 |
| Energy | | 344.6 | 345.5 | 358.0 |
| Ascorbic acid (100% pure) | | | | 0.16 g |
| Vit. E (Tocopherol acetate, 50 %) | | | | 0.465 g |
| Vitamine D (cholecalciferol, 500.000 IU/g) | | | | 5.6 µg |
| Vit. B6 (Pyridoxin hydrochloride, 82 %) | | | | 0.00407 g |
| Folic acid (100%) | | | | 0.00060 g |
| Vit. B12 (Cyanocobalamin 0,1 %) | | | | 0.0575 g |
| Na selenite • 5 H2O | | | | 0.00034 g |

| | | | | |
|---|---|---|---|---|
| ¹ Butyrate producing fibres (5 gram per 100 g diet): 1.50 g GOS 0.17 g long-chain FOS 1.67 g short- chain FOS 1.67 g nutriose And further 0.5 g lactose and 0.5 g the carbohydrate and moisture to 7.27 g ² Additional vitamins (per 100 g diet) | | | | |

Diet 1 was composed of the control diet plus additionally UMP and DHA (fish oil).

Diet 2 was as Diet 1, without cellulose , but instead additionally a mixture of galactooligosaccharides and fructooligosaccharides (both long chain and short chain fructooligosaccharides and instead of UMP uridine.

A rotarod test was performed for 10 weeks, starting 1 week after intra-striatal injection with rotenone or sham. As shown in Figure 2, rotenone induced motor problems, but after start of treatment with Diet 1 or Diet 2, an improvement in motoric functioning was shown (increased average time on the rod, compared to the 'Rotenone + Control Food' group). The additional presence of the dietary fibre had an positive effect on motoric functioning.

## Claims

1. A combination of a uridine source selected from the group of uridine, deoxyuridine, cytidine, deoxycytidine, UMP, UDP, UTP, CMP, CDP, CTP, dUMP, dUDP, dUTP, dCMP, dCDP and dCTP, wherein optionally one or more hydroxyl moieties of the (deoxy)ribose of said uridine source are acylated with a C1-C24 carboxylic acid, and an omega-3-polyunsaturated fatty acid having 18-24 carbon atoms selected from the group consisting of docosahexaenoic acid (DHA), docosapentaenoic acid (DPA) and eicosapentaenoic acid (EPA) for a use selected from the group consisting of
- the prevention or treatment of a disturbance in coordination of limbs in a mammal;
- the prevention or treatment of a disturbance in equilibrium in a mammal;
- the prevention or treatment of a disturbance in limb strength, in particular forelimb grip strength in a mammal;
- the prevention or treatment of a disturbance in a fine-motor skill in a mammal; and
- the prevention or treatment of a disturbance in a gross-motor skill in a mammal,
wherein the combination further comprises at least one component from the group of vitamin B6, vitamin B9 and vitamin B12 and/or the combination further comprises vitamin D and/or the combination further comprises an antioxidant selected from the group of vitamin C, vitamin E and selenium and/or the combination is to be administered as part of a nutritional composition further comprising a phospholipid and/or the combination is to be administered as part of a nutritional composition containing a choline and/or the combination is to be administered as part of a nutritional composition comprising a protein.

2. A combination for use according to claim 1, wherein the mammal suffers from a neurological disorder, preferably a peripheral neuropathy, an autonomic neuropathy, an enteric nervous system neuropathy, a synucleopathy, diabetic neuropathy, Duchenne's dystrophy, cerebrovascular disease, Multiple Sclerosis, pure autonomic failure spinal cord injury, cerebrovascular disease, Multiple Sclerosis, pure autonomic failure, spinal cord injury, Parkinson's disease or a dementia, which dementia preferably is Alzheimer Disease.

3. A combination for use according to claim 1, wherein the mammal suffers from a psychiatric disorder, which psychiatric disorder preferably is pervasive development disorder, more preferably autistic spectrum disorder, or wherein the psychiatric disorder is depression, in particular a depressive mood disorder.

4. A combination for use according to any of the preceding claims, comprising DHA and optionally EPA, wherein the daily dosage of DHA plus EPA administered via the combination is in the range of 400 mg to 7500 mg, preferably in the range of 500 mg to 5000 mg, in particular in the range of 1000 mg to 3000 mg.

5. A combination for use according to any of the preceding claims, wherein the uridine source is selected from the group consisting of non-acylated UMP and acylated UMP's or, wherein the uridine source comprises a nucleoside selected from the group of uridine and deoxyuridine, wherein optionally one or more hydroxyl moieties of the (deoxy)ribose of said nucleoside are acylated.

6. A combination for use according to any of the preceding claims, wherein the daily dosage of the uridine source is in the range of 1-150 µmol per kg body weight per day, preferably in the range of 3-116 µmol per kg body weight per day.

7. A combination for use according to any of the preceding claims, wherein the combination comprises a dietary fibre, preferably a dietary fibre selected from the group of butyrate producing fibres, more preferably at least one butyrate producing fibre is present selected from the group of fructooligosaccharides (FOS), galactooligosaccharides (GOS) and dextrins (e.g. Nutriose®).

8. A combination for use according to any of the preceding claims, , wherein the combination is to be administered as part of a nutritional composition, comprising at least one component selected from the group of vitamin E, vitamin C and selenium.

9. A combination for use according to any of the preceding claims, wherein the combination comprises at least one vitamin selected from the group of vitamin B12, vitamin B6 and folic acid, preferably comprising vitamin B12, vitamin B6 and folic acid.

10. A combination for use according to claim 9 wherein the combination is part of a nutritional composition, the nutritional composition comprising
i-) at least one component selected from the group of uridine, acylated uridine, uridine monophosphate and acylated uridine monophosphate.
ii) DHA and EPA
iii) a vitamin B selected from the group of vitamin B6, vitamin B9 and vitamin B12
iv) a phospholipid
v) an antioxidant selected from the group of vitamin C, vitamin E and selenium
vi) a choline
vii) a protein.

11. A combination for use according to any of the preceding claims, wherein the combination comprises vitamin D, preferably vitamin D3.

12. A combination for use according to claim 11, wherein vitamin D, preferably vitamin D3, is to be administered in a daily dosage of 5-50 µg, in particular 25-40 pg.

13. A combination for use according to any of the preceding claims, wherein the mammal is a human, preferably a human of at least 18 years of age, in particular an elderly human.

14. Non-medical use of a combination of a uridine source and an omega-3-polyunsaturated fatty acid having 18-24 carbon atoms for improving coordination of limbs in a mammal, for improving sense of equilibrium in a mammal, for improving forelimb grip strength in a mammal, for improving a fine-motor skill in a mammal or for improving a gross-motor skill, wherein the combination is as defined in any of the claims 4-13.
